# EUROPEAN PATENT APPLICATION

(11) **EP 3 669 756 A1**
(43) Date of publication of application: **24.06.2020**
(21) Application number: 18212971.8
(22) Date of filing: 17.12.2018
(51) Int. Cl.: A61B 5/00, A61B 5/01, A61B 5/024, A61B 5/026, A61B 5/107

(54) **DEVICE, SYSTEM AND METHOD FOR DETERMINING A VITAL SIGN OF A SUBJECT**

(71) Applicant: Koninklijke Philips N.V., 5656 AG Eindhoven (NL)
(72) Inventor: VERKRUIJSSE, Willem, 5656 AE Eindhoven (NL); MENA BENITO, Maria Estrella, 5656 AE Eindhoven (NL); MEFTAH, Mohammed, 5656 AE Eindhoven (NL)
(74) Representative: de Haan, Poul Erik

(57) **Abstract**

The present invention relates to a device, system and method for determining a vital sign of a subject. To increase PPG signal strength in remote vital signs monitoring without compromising the unobtrusiveness the device comprises an input unit (31) configured to obtain a detection signal derived from contactlessly detected electromagnetic radiation transmitted through or reflected from a skin region of a subject, a vital sign determination unit (32) configured to determine a vital sign from said detection signal, and a control unit (33) configured to generate a control signal for controlling a stimulation unit (10) configured to locally stimulate a skin region of the subject to cause vasodilation of the stimulated skin region.

## Description

### FIELD OF THE INVENTION

The present invention relates to a device, system and method for determining a vital sign of a subject.

### BACKGROUND OF THE INVENTION

Vital signs of a person, for example the heart rate (HR), the respiration rate (RR) or the (peripheral or pulsatile) blood oxygen saturation (SpO2; it provides an estimate of the arterial blood oxygen saturation SaO2), serve as indicators of the current state of a person and as powerful predictors of serious medical events. For this reason, vital signs are extensively monitored in inpatient and outpatient care settings, at home or in further health, leisure and fitness settings.

One way of measuring vital signs is plethysmography. Plethysmography generally refers to the measurement of volume changes of an organ or a body part and in particular to the detection of volume changes due to a cardio-vascular pulse wave traveling through the body of a subject with every heartbeat.

Photoplethysmography (PPG) is an optical measurement technique that evaluates a time-variant change of light reflectance or transmission of an area or volume of interest. PPG is based on the principle that the blood absorbs light more than the surrounding tissue, so variations in blood volume with every heartbeat affect the transmission or reflectance correspondingly. Besides information about the pulse rate (heart rate), a PPG waveform (also called PPG signal) can comprise information attributable to further physiological phenomena such as the respiration. By evaluating the transmittance and/or reflectance at different wavelengths (typically red and infrared), the blood oxygen saturation can be determined.

Non-contact, remote PPG (rPPG) devices (also called camera-based devices) for unobtrusive measurements have been proposed in the last decade. Remote PPG utilizes light sources or, in general, radiation sources, disposed at a distance from the subject of interest. Similarly, a detector, e.g. a camera or a photodetector, can be disposed at a distance from the subject of interest. Therefore, remote photoplethysmographic systems and devices are considered unobtrusive and well suited for medical as well as non-medical everyday applications.

Using the PPG technology, vital signs can be measured. Vital signs are revealed by minute light absorption changes in the skin caused by the pulsating blood volume, i.e. by periodic color changes of the human skin induced by the blood volume pulse. As this signal is very small and hidden in much larger variations due to illumination changes and motion, there is a general interest in improving the fundamentally low signal-to-noise ratio (SNR). The measurement of one of the important vital signs, the blood oxygen saturation (SpO2), has recently shown to be feasible with camera-based rPPG. SpO2 measurements require an accurate detection of relative pulsatilities, i.e. the normalized amplitude of the pulsatile signal (AC/DC) in different wavelength channels.

Many patients with fragile skin (e.g. NICU (neonatal intensive care unit) patients) would greatly benefit from contactless monitoring of vital signs. RPPG) devices for unobtrusive measurements of pulse, respiration, SpO2, etc. are being developed and have been disclosed in several publications. The principle of rPPG depends on small modulations in skin reflectance. A disadvantage of rPPG compared to conventional contact PPG is that the remote PPG signal strength is inherently much lower (about 1 - 2 orders of magnitude). Moreover, in some patients this signal can be very small due to their physiological status, e.g. when they are centralized. While on adult patients rPPG can be very difficult when the 'hot spots' for rPPG are covered by bandages for example, in neonates, the signal is small overall. If the PPG signal is small, the SNR required for rPPG based monitoring (of SpO2, for example) can thus easily drop below measurement thresholds and endanger continuous monitoring.

A successful introduction of remote pulse rate and SpO2 in the NICU and in adult centralized (low pulsatility) patients may need an increase of PPG signal strength. Hence, there is a need for a solution that increases the PPG signal strength in remote (camera-based) vital signs monitoring without compromising the unobtrusiveness of this sensing technique.

### SUMMARY OF THE INVENTION

It is an object of the present invention to provide a device, system and method for determining a vital sign of a subject with increased PPG signal strength in remote vital signs monitoring without compromising the unobtrusiveness.

In a first aspect of the present invention a device for determining a vital sign of a subject is presented comprising
- an input unit configured to obtain a detection signal derived from contactlessly detected electromagnetic radiation transmitted through or reflected from a skin region of a subject,
- a vital sign determination unit configured to determine a vital sign from said detection signal, and
- a control unit configured to generate a control signal for controlling a stimulation unit configured to locally stimulate a skin region of the subject to cause vasodilation of the stimulated skin region.

In a further aspect of the present invention a system for determining a vital sign of a subject is presented comprising
- a stimulation unit configured to locally stimulate a skin region of the subject to cause vasodilation of the stimulated skin region,
- a detection unit configured to contactlessly detect electromagnetic radiation from the stimulated skin region and/or to derive the detection signal from electromagnetic radiation detected from the stimulated skin region, and
- a device as disclosed herein configured to determine a vital sign of the subject from said detection signal.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed system, in particular as defined in the dependent claims and as disclosed herein.

The present invention is based on the idea to increase the pulsatile component of the reflected or transmitted electromagnetic radiation and, hence, in a detection signal (such as a photoplethysmography (PPG) signal corresponding to or derived from the detection signal) evaluated for determining a vital sign by stimulating the skin and/or tissue. Stimulation of all visible skin surface at once is undesired for the risk of overheating the patient so that heating of skin shall be local (e.g. at skin regions of a size in the range of 3-5 cm²). Furthermore, the local heating shall be temporary (e.g. for a duration in the range of 1-30 minutes) to prevent undesired transient effects and local overheating. The proposed stimulation, therefore, is applied both locally and temporarily to increase signal strength locally by stimulating vasodilation of skin areas for a predetermined time. Afterwards, the stimulation may, as provided in an embodiment, automatically switch to another skin region to avoid overstimulation. The present invention thus provides an automated solution that safely increases the PPG signal strength to enable continuous rPPG monitoring without compromising the unobtrusiveness and contactless nature of the remote monitoring.

In an embodiment the control unit is configured to generate a control signal for controlling location, duration and/or intensity of the stimulation. For each location the duration and/or intensity may be controlled separately and differently to optimize the stimulation and the result of the stimulation.

The control unit may further be configured to generate a control signal for controlling a detection unit configured to contactlessly detect electromagnetic radiation from the stimulated skin region and/or to derive the detection signal from electromagnetic radiation detected from the stimulated skin region and/or for controlling the vital sign determination unit to determine the vital signal from a detection signal derived from contactlessly detected electromagnetic radiation transmitted through or reflected from a stimulated skin region. This ensures that when the location of the vital sign is changed the electromagnetic radiation from the new location is automatically used to determine the vital sign. This further ensures that an automatic continuous monitoring can be established.

According to another embodiment the control unit is configured to generate a control signal for controlling the stimulation unit to find another skin region of the subject to stimulate based on one or more of a distance to the previously stimulated skin region, the temperature of different skin regions, the location, orientation and/or size of different skin regions, motion of the subject, responsiveness of previously stimulated skin regions to stimulation, and the intensity of the detection signal and/or a photoplethysmography, PPG, signal derived from the detection signal. This avoids overheating, enables continuous monitoring and optimizes the results of the vital sign determination to obtain a reliable and robust vital sign.

The control unit may further be configured to generate a control signal for controlling the intensity of the stimulation by the stimulation unit based on the intensity of the detection signal and/or a photoplethysmography, PPG, signal derived from the detection signal. The use of this feedback control ensures a sufficiently strong detection signal or PPG signal.

In another embodiment the control unit is configured to generate a control signal for controlling location of the stimulation based on detection of an alignment signal emitted by the stimulation unit and detected by a detection unit configured to contactlessly detect electromagnetic radiation. This ensures that the desired location is stimulated.

The control unit may be configured to generate a control signal for controlling location of the stimulation based on the intensity of the detection signal and/or a photoplethysmography, PPG, signal derived from the detection signal. For instance, an intensity map may be created from electromagnetic radiation detected from the complete field of view of the detection unit (e.g. a camera). A spot in the intensity map showing an increased intensity (compared to other regions) then indicates the skin region (also called 'spot' herein) that is stimulated.

In another embodiment the control unit is configured to generate a control signal for controlling the intensity of the stimulation by the stimulation unit based on the angle of the skin with respect to the stimulation unit. For instance, 3D information of the scene may be obtained, e.g. by a depth camera or time-of-flight (TOF) camera, to determine the angle of the skin with respect to the stimulation so that the intensity of the stimulation may be adjusted accordingly.

The device may further comprise a recognition unit configured to recognize body parts of the subject that lack vasodilatory nerves, wherein the control unit is configured to generate a control signal for controlling the stimulation unit not to locally stimulate skin regions of said body parts that lack vasodilatory nerves. Glabrous skin on those body parts (e.g. foot soles, hand palms, lips, etc.) can thus be excluded from the stimulation.

The control unit may further be configured to generate a control signal for controlling two or more locations of partly simultaneous stimulation by the stimulation unit, wherein stimulation of a second location is started before stimulation of a first location is stopped. This further supports a continuous monitoring of a subject without any interruptions.

The claimed system comprises, besides the above described device, a stimulation unit for locally stimulating a skin region of the subject to increase the skin temperature of the stimulated skin region and a detection unit for contactlessly detecting electromagnetic radiation from the stimulated skin region and/or deriving the detection signal from electromagnetic radiation detected from the stimulated skin region.

The stimulation unit may be configured to stimulate the skin region to cause vasodilation by a bio-thermal, bio-chemical, and/or photo-biological mechanism. The stimulation may thus be effected by heat (e.g. an air flow), light (e.g. a laser beam), electromagnetic radiation (e.g. infrared light) or in any other (preferably contactless) way that locally stimulates the skin region so that it causes vasodilation leading to an increased PPG signal strength in the stimulated skin region. In one embodiment thermal stimulation is applied, but in other embodiments light is used for stimulation, in particular blue light that is more effective than other colors. In still another embodiment a fluid spray, such as a spray containing capsaicin (tiger balm or pepper) may be applied by the stimulation unit onto the skin region to enhance PPG signal strength temporarily.

The detection unit may be a single pixel detector (e.g. a photodetector) or a camera (e.g. a video camera, such as an RGB camera or a near-infrared (NIR) camera).

The system may further comprise an actuator configured to change the direction and/or location of the stimulation. The actuator may be configured to directly manipulate the stimulation unit (e.g. by use of a motor to change the direction of emitted radiation or heat) or to indirectly change the direction of the stimulation (e.g. by use of a mirror to change the direction of emitted radiation).

The system may still further comprise a thermal sensor configured to monitor one or more of the location of the stimulation, the intensity of the stimulation, the duration of the stimulation and the skin temperature of the stimulated skin region. Such a (preferably contactless) thermal sensor (e.g. a thermal-infrared sensor or camera) enables monitoring of the effect of the stimulation, ensures safety by avoiding overheating of a skin region and supports the finding of skin regions to stimulate.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of a first embodiment of a system according to the present invention,
Fig. 2 shows a schematic diagram of a first embodiment of a device according to the present invention,
Fig. 3 shows a diagram illustrating the adaptation of the intensity of the stimulation based on the skin angle,
Fig. 4 shows a schematic diagram of a second embodiment of a system according to the present invention,
Fig. 5 shows a schematic diagram of a third embodiment of a system according to the present invention,
Fig. 6 shows a schematic diagram of a second embodiment of a device according to the present invention, and
Fig. 7 shows a diagram illustrating the effect of local warming of a skin area on the cutaneous vascular conductance.

### DETAILED DESCRIPTION OF EMBODIMENTS

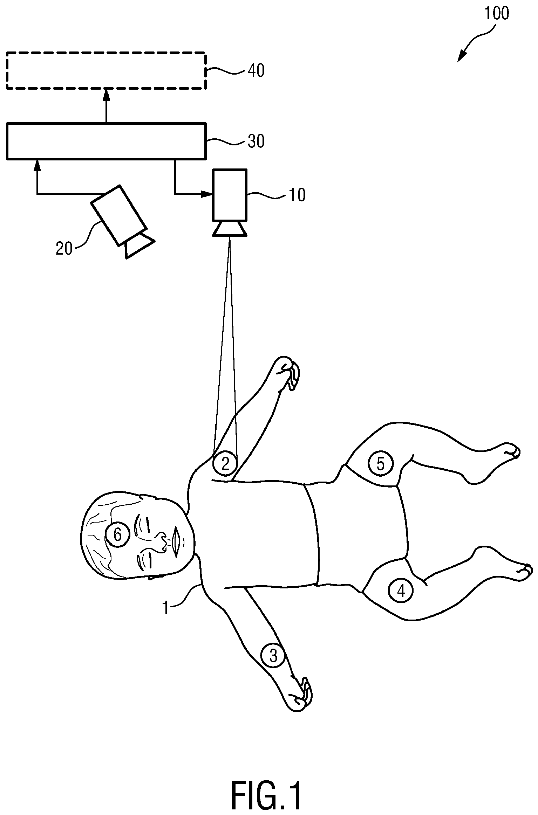

Fig. 1 shows a schematic diagram of a first embodiment of a system 100 according to the present invention. The system 100 comprises a stimulation unit 10 configured to locally stimulate a skin region 2 (or any one of the different skin regions 3 to 6) of the subject 1 to cause vasodilation of (or in) the stimulated skin region 2. The system 100 further comprises a detection unit 20 configured to contactlessly detect electromagnetic radiation from the stimulated skin region 2 and/or to derive the detection signal from electromagnetic radiation detected from the stimulated skin region 2. Still further, the system 100 comprises a device 30 configured to determine a vital sign of the subject 1 from said detection signal. The subject 1, in this example an infant, may lie in a bed, e.g. in a hospital or other healthcare facility, but may also be a neonate or premature infant lying in an incubator, or person at home or in a different environment.

The stimulation unit 10 may be configured as radiative and/or convective stimulation source that stimulates a skin region (in a contactless manner) to locally cause vasodilation, e.g. to locally increase the skin temperature causing vasodilation, to enhance the PPG amplitude in targeted skin areas for a predefined time. Local warming of the skin may preferably be done contactlessly by emitting light (e.g. by laser or broadband light sources or blue light), and/or heat waves (thermal infrared, IR), and/or a warm air flow. Vasodilation may further be caused by the application of a fluid spray (or even ointment) containing capsaicin or by other means effecting a bio-chemical or photo-biological mechanism.

The detection unit 20 may include a single- or multi-pixel photodetector or a camera (also referred to as imaging unit, or as camera-based or remote PPG sensor, vital signs camera (VSC) or rPPG camera) including a suitable photosensor for (remotely and unobtrusively) capturing image frames of the subject 1, in particular for acquiring a sequence of image frames of the subject 1 over time, from which PPG signals can be derived. The detection unit 20 is preferably configured to detect electromagnetic radiation in the visible and/or near-infrared spectral range at one or more wavelengths for continuous non-contact monitoring and measurement of physiological signals (vital signs).

The image frames captured by the camera may particularly correspond to a video sequence captured by means of an analog or digital photosensor, e.g. in a (digital) camera. Such a camera usually includes a photosensor, such as a CMOS or CCD sensor, which may also operate in a specific spectral range (visible, IR) or provide information for different spectral ranges. The camera may provide an analog or digital signal.

The image frames include a plurality of image pixels having associated pixel values. Particularly, the image frames include pixels representing light intensity values captured with different photosensitive elements of a photosensor. These photosensitive elements may be sensitive in a specific spectral range (i.e. representing a specific color or pseudo-color (in NIR)). The image frames include at least some image pixels being representative of a skin portion of the subject. Thereby, an image pixel may correspond to one photosensitive element of a photo-detector and its (analog or digital) output or may be determined based on a combination (e.g. through binning) of a plurality of the photosensitive elements.

The device 30 may be configured as digital or analog processor depending on how and where the invention is applied. The functions of the device 30 may completely or partly be implemented in software and carried out on a personal computer connected to the detection unit 20 (and optionally the stimulation unit). Some or all of the required functionality may also be implemented in hardware, e.g. in an application specific integrated circuit (ASIC) or in a field programmable gate array (FPGA).

The device 30 may further be connected to an interface 40 for displaying the determined information and/or for providing medical personnel with an interface to change settings of the device 30, the detection unit 20 and/or the stimulation unit 10. Such an interface 40 may comprise different displays, buttons, touchscreens, keyboards or other human machine interface means.

A system 100 as illustrated in Fig. 1 may, e.g., be located in a hospital, healthcare facility, elderly care facility or the like. Apart from the monitoring of patients, the present invention may also be applied in other fields such as neonate monitoring, general surveillance applications, security monitoring or so-called lifestyle environments, such as fitness equipment, a wearable, a handheld device like a smartphone, or the like. The uni- or bidirectional communication between the device 30, the detection unit 20, the stimulation unit 10 and the optional interface 40 may work via a wireless or wired communication interface. Other embodiments of the present invention may include a device 30, which is not provided stand-alone, but integrated into the detection unit 20 or the interface 40.

Typically, the electromagnetic radiation detected by the detection unit 20 is in the range of 400 nm to 1000 nm for pulse, respiration and blood oxygen saturation measurement, particularly in the range of 620 nm to 920 nm. This particular range is most suitable for SpO2 measurement and is attractive for unobtrusive monitoring during sleep (darkness), but if pulse or respiratory signals are required, the visible part of the spectrum may allow a higher quality (i.e. NIR is not necessarily the preferred option in all cases).

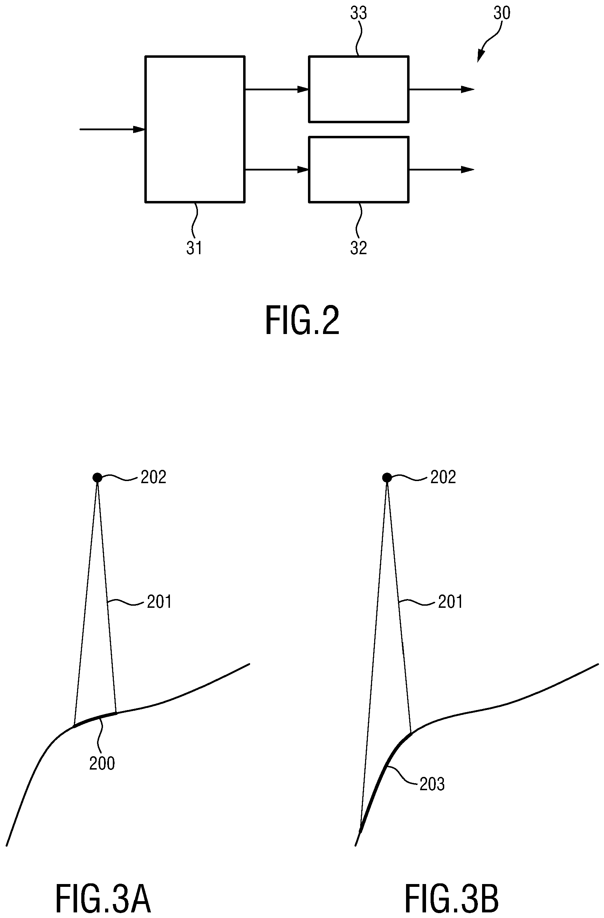

Fig. 2 shows an embodiment of a device 30 for determining a vital sign of a subject 1 according to the present invention. The device 30 comprises an input unit 31 configured to obtain a detection signal derived from contactlessly detected electromagnetic radiation transmitted through or reflected from a skin region of a subject 1. The device 30 further comprises a vital sign determination unit 32 configured to determine a vital sign from said detection signal. Still further, the device 30 comprises a control unit 33 configured to generate a control signal for controlling the stimulation unit 10 to locally stimulate a skin region 2 of the subject 1 to increase the skin temperature of the stimulated skin region 2.

Thus, even if a patient has insufficient signal strength of the detection signal or a PPG signal, local stimulation is applied according to the present invention to increase signal strength is provided to enable continuous rPPG based monitoring. This is done automatically according to the present invention which is much less cumbersome compared to manual stimulation, which is practically impossible when continuous monitoring is required.

Stimulation of all skin is undesired because it may disturb the thermoregulation (which is undeveloped in neonates) and overheating of the patient. Therefore, a spot-based and temporary stimulation is provided according to the present invention. However, even when a small spot is stimulated, regulation of stimulation time and stimulation intensity is preferably controlled because of dehydration of the stimulated skin and a 'die away' effect of thermal stimulation. For this reason, the stimulation is switched automatically from time to time from one skin region to another skin region, which is efficient and safe.

According to the present invention no hand-held application of stimulation is required, which is not possible with current workflows for continuous monitoring. Further, the present invention makes it possible to keep track of which skin regions were recently stimulated and to find a new skin region for stimulation. Still further, disabilities of a patient, such as a tremor in the hand holding the stimulation unit, do not negatively impact efficient and controlled stimulation of the skin region (e.g. with manual stimulation more tremor would cause a larger spot and hence less irradiation/cm²).

In an embodiment of the present invention, a skin map, i.e. visible skin, is determined, based on which a skin region to be stimulated is selected. The stimulation unit is navigated to the selected skin region (i.e. controlled to stimulate the selected skin region) and is activated to start the stimulation. The second and third steps may be repeated for new skin regions to be stimulated.

To determine a skin map various techniques may be used: using skin color, living pixels, or thermal information (skin is typically warmer than textiles and other objects). This technique may require a PPG signal, which may be still insufficient for SpO2 determination. The 'living pixels' technique may use a PPG signal in the green wavelength range, with a strong signal, so that they may be good for 'living pixel' skin detection. A green PPG signal is however not preferred for SpO2 determination so that stimulation for SpO2 would still be necessary as contrast for SpO2 is at red wavelengths (e.g. 660 nm), where signal strength is very low. Further, pattern and face recognition may be applied so that the skin area around the eyes can be excluded from the skin map for safety reasons. Similarly, in the case of NICU applications, skin near contact sensors to probe (core-) temperature are excluded in order to not disrupt this thermal measurement.

A criterion for the selection of a new skin region to be stimulated may include that a new skin region should be sufficiently far away from the previous skin region to avoid overlapping of vasodilatory effects since it is known that the effects of local heating may spread beyond the stimulated area. Other criteria may help to determine the next skin region, e.g. temperature, location/orientation/size, motion, responsiveness to stimulation, initial signal strength of the detection signal or a PPG signal.

If light is used for stimulation (instead of thermal IR or warm air) care should be taken that wavelengths used do not interfere with wavelengths used for determining the vital sign from the detection signal. For example, vital signs cameras may use wavelengths from red to about 950 nm. Wavelength bands in this range may be used for stimulation, as long as they are not used for PPG to determine the vital sign.

It shall be noted that in the embodiment of the system 100 shown in Fig. 1 and device 30 shown in Fig. 2 the temperatures (and increases resulting from stimulation) are not known since there is no thermal camera used. Therefore, to ensure safety, the maximum stimulation intensities and the resulting increased perfusion are preferably limited. Nevertheless, this embodiment works and improves rPPG monitoring.

In another embodiment the local signal strength of the detection signal or the PPG signal (on the skin region that is being stimulated) is used as feedback to the stimulus intensity, i.e. for the control by the control unit 33. The intensity of stimulation (within the safety limits) may thus automatically adapt to ensure a sufficiently strong detection signal and/or PPG signal (using a feedback control mechanism).

The stimulation unit 10 and the detection unit 20 will likely have some parallax (i.e., the stimulation beam of the stimulation unit 10 and the viewing beam of the detection unit 20) do not have the same origin and do not coincide). Alignment of the stimulation beam with the detection unit 20 may be provided (e.g. when the distance between the stimulation unit / detection unit and the subject has changed), or simply after installation of the system 100. Alignment of the stimulation spot with the images from a camera (as the detection unit 20) may be done by mixing a small amount of light in the stimulation for which the camera is sensitive. This allows for self-alignment, which may be done prior to the actual start of the stimulation.

Alternatively, self-alignment may be done using PPG imaging. A stimulation starts with moderate intensity and the device 30 creates a PPG amplitude map and finds the skin region that is stimulated by observing increased signal strength of the detection signal or the PPG signal, or by looking at a decreased green level (more blood) using e.g. an RGB camera.

The effect of stimulation of a skin region with radiation (light or thermal radiation) depends quite strongly on the angle of the skin with respect to the stimulation as illustrated in Fig. 3. This figure particularly shows that the irradiance depends on orientation of skin and distance to the subject. With a 3D camera e.g. a time of flight camera, the stimulation intensity can be adjusted to account for this effect. Fig. 3A shows a situation where the irradiated skin region 200 is arranged almost perpendicularly to the radiation beam 201 of the stimulation source 202. Fig. 3B shows a situation where the irradiated skin region 203 is arranged at a much smaller angle to the radiation beam 201 of the stimulation source 202. This has the consequence that - provided the power of the emitted radiation is the same (e.g. 1 W) in both situations - that the irradiated skin region is much larger in the situation shown in Fig. 3B and that hence the power per cm² is much smaller (e.g. 2 W / cm²) in this situation compared to the situation shown in Fig. 3A (having an irradiance of e.g. 5 W / cm²).

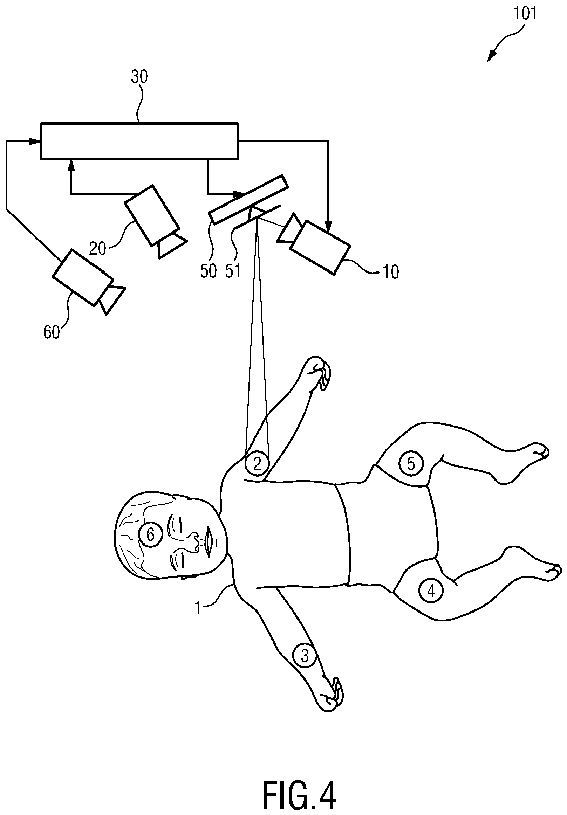

In this embodiment 3D information from a TOF camera may be used to estimate the angle of the skin and adjust the stimulation intensity proportionally. An embodiment of the system 101 comprising such an addition TOF camera 60 is shown in Fig. 4. This is an improvement over the above described embodiment in the sense that it will allow for correcting for the effect described above and avoid very low stimulation intensities that may barely cause the desired effect of vasodilation and increased PPG signal. If illumination/stimulation angles are too shallow, the skin region will not be used for vital signs determination because the illumination becomes impractical.

In the embodiment of the system 101 shown in Fig. 4 an actuator 50 is provided to change the direction of the stimulation, e.g. of the stimulation beam emitted by the stimulation unit 10 in order to move the stimulation from one skin region to another. The actuator 50 (e.g. a motor or manipulator) may be directly coupled to the stimulation unit 10 in order to change the orientation of the stimulation unit 10 beam, or may be coupled to a mirror 51, as shown in Fig. 4, which changes the direction of the stimulation beam and which allows for a smaller mechanical load on the actuator 50.

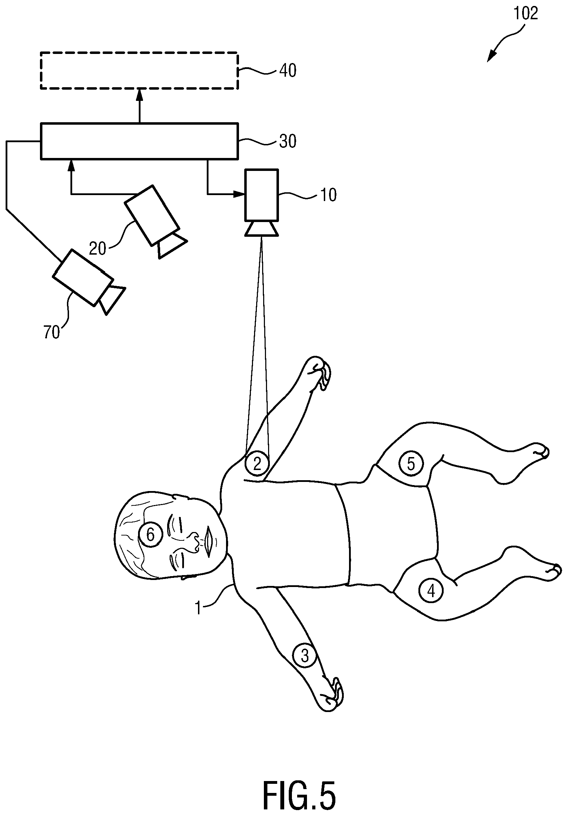

The 3D solution using a TOF camera 60 may not be necessary if a thermal camera can provide feedback on the stimulation efficacy as provided in another embodiment of the system 102 shown in Fig. 5. In this embodiment a thermal sensor 70 (preferably a thermal camera; a guided single element thermal sensor can be used as well) to provide feedback to the control unit 33 of the device 30. The thermal sensor 70 has three functions: to ensure safety (so that temperatures do not exceed predefined threshold temperatures), to control illumination intensity/efficacy, and to provide information to help choosing the next skin region.

Safety is straightforward since skin temperature should not exceed those at which pain/discomfort and/or skin damage occur.

Controlling illumination intensity/efficacy is an important improvement over embodiments without a thermal camera. In these embodiments, the stimulation intensity is kept on the safe side as there is no feedback on the actual skin temperature (although the PPG signal strength for feedback on stimulation efficacy is used in one embodiment). This embodiment improves by using information on the actual skin temperatures, allowing for higher stimulation intensities (and stronger PPG signals) without compromising safety. Obviously, also in this embodiment the maximum stimulation intensity is restricted and within predetermined safe ranges.

Providing information allows for smarter selection of next skin regions to stimulate based on the skin temperature map. For example, a skin region with a low temperature is likely to have not been stimulated for some time such that it may receive a higher priority than a skin region with relatively high temperature.

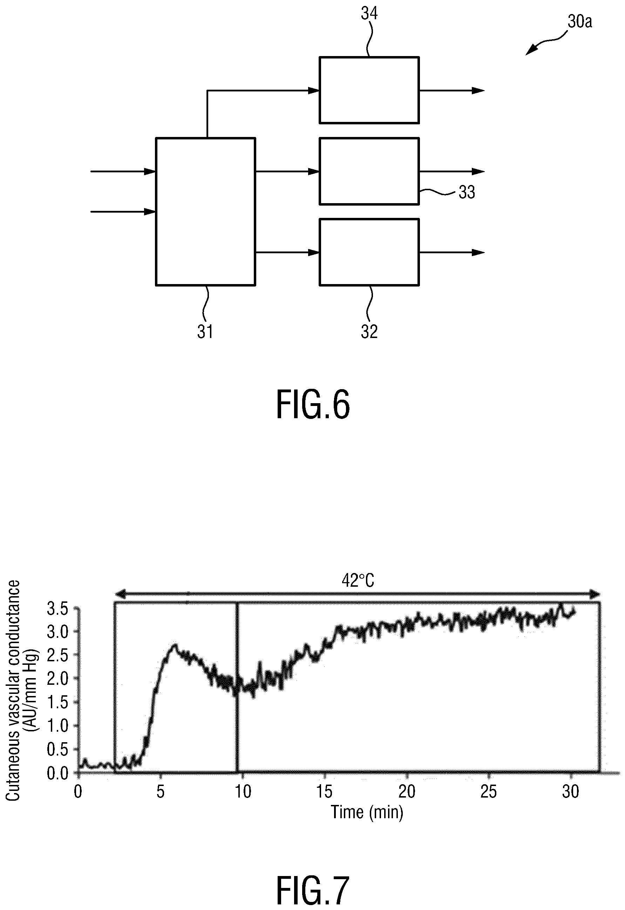

In another embodiment a (normal) RGB camera and/or NIR camera (for low illumination conditions) may be used to provide input to a pattern recognition unit 34, as shown in another embodiment of the device 30a depicted in Fig. 6, to recognize certain body parts that lack vasodilatory nerves and will therefore not respond to thermal stimulation. Skin on such body parts is called glabrous skin. Foot soles, hand palms, lips have glabrous skin areas and should be excluded from stimulation.

In another embodiment, the stimulation unit may be configured to emit two (or more) stimulation beams. One of the beams is used to stimulate the current skin region (spot A) that serves to provide the PPG based measurement, while another beam is used to preheat one or more next skin regions (spot B, spot C, ...) to be used for subsequent PPG based measurement. This preheating may start at a certain time (e.g. one or two minutes) before the measurement on spot A ends. By doing so, it is avoided that after switching to a new skin region, the new skin region has insufficient vasodilation/PPG signal, thus enabling continuous PPG monitoring.

Fig. 7 shows a diagram that illustrates this effect. Shown is the cutaneous vascular conductance 300 over time (during 30 minutes of warming). In a first phase 301 a rapid peak appears due to local sensory nerve activity. The second (slower) phase 302 depends on the nitric oxide. Hence, the optimal perfusion is only reached some minutes after stimulation starts.

In the case of moderate motion (e.g. respiration) the effective stimulation spot is larger than if the skin would be motionless. In such cases the stimulation intensity may be adjusted accordingly to result in an irradiance as if skin were motionless. A thermal feedback (from the thermal sensor 70) may be used for this adjustment.

Currently, tracking of skin regions with few and weak features is difficult. However, if tracking is possible anyway, the stimulation beam will track the illumination spot.

The present invention extends the value of camera-based patient-monitors for patient monitoring. The contactless monitoring, preferably with a camera, is assumed to be highly relevant for premature babies with very sensitive skin in NICUs, patients with damaged (e.g. burnt) skin, adult patients who have their PPG 'hot spots' covered by bandages or other medical equipment/objects, etc. The present invention is not limited to applications in the NICU. It can be applied in other use cases as well, e.g. in centralized adult patients.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device for determining a vital sign of a subject, said device comprising:
- an input unit (31) configured to obtain a detection signal derived from contactlessly detected electromagnetic radiation transmitted through or reflected from a skin region of a subject,
- a vital sign determination unit (32) configured to determine a vital sign from said detection signal, and
- a control unit (33) configured to generate a control signal for controlling a stimulation unit (10) configured to locally stimulate a skin region of the subject to cause vasodilation of the stimulated skin region.

2. Device as claimed in claim 1,
wherein the control unit (33) is configured to generate a control signal for controlling location, duration and/or intensity of the stimulation.

3. Device as claimed in any one of the preceding claims,
wherein the control unit (33) is configured to generate a control signal for controlling a detection unit (20) configured to contactlessly detect electromagnetic radiation from the stimulated skin region and/or to derive the detection signal from electromagnetic radiation detected from the stimulated skin region and/or for controlling the vital sign determination unit (32) to determine the vital signal from a detection signal derived from contactlessly detected electromagnetic radiation transmitted through or reflected from a stimulated skin region.

4. Device as claimed in any one of the preceding claims,
wherein the control unit (33) is configured to generate a control signal for controlling the stimulation unit (10) to find another skin region of the subject to stimulate based on one or more of a distance to the previously stimulated skin region, the temperature of different skin regions, the location, orientation and/or size of different skin regions, motion of the subject, responsiveness of previously stimulated skin regions to stimulation, and the intensity of the detection signal and/or a photoplethysmography, PPG, signal derived from the detection signal.

5. Device as claimed in any one of the preceding claims,
wherein the control unit (33) is configured to generate a control signal for controlling the intensity of the stimulation by the stimulation unit (10) based on the intensity of the detection signal and/or a photoplethysmography, PPG, signal derived from the detection signal.

6. Device as claimed in any one of the preceding claims,
wherein the control unit (33) is configured to generate a control signal for controlling location of the stimulation based on detection of an alignment signal emitted by the stimulation unit and detected by a detection unit (20) configured to contactlessly detect electromagnetic radiation.

7. Device as claimed in any one of the preceding claims,
wherein the control unit (33) is configured to generate a control signal for controlling location of the stimulation based on the intensity of the detection signal and/or a photoplethysmography, PPG, signal derived from the detection signal.

8. Device as claimed in any one of the preceding claims,
wherein the control unit (33) is configured to generate a control signal for controlling the intensity of the stimulation by the stimulation unit (10) based on the angle of the skin with respect to the stimulation unit.

9. Device as claimed in any one of the preceding claims,
further comprising a recognition unit (34) configured to recognize body parts of the subject that lack vasodilatory nerves,
wherein the control unit (33) is configured to generate a control signal for controlling the stimulation unit (10) not to locally stimulate a skin regions of said body parts that lack vasodilatory nerves.

10. Device as claimed in any one of the preceding claims,
wherein the control unit (33) is configured to generate a control signal for controlling two or more locations of partly simultaneous stimulation by the stimulation unit, wherein stimulation of a second location is started before stimulation of a first location is stopped.

11. System for determining a vital sign of a subject, said system comprising:
- a stimulation unit (10) configured to locally stimulate a skin region of the subject to cause vasodilation of the stimulated skin region,
- a detection unit (20) configured to contactlessly detect electromagnetic radiation from the stimulated skin region and/or to derive the detection signal from electromagnetic radiation detected from the stimulated skin region, and
- a device (30, 30a) according to any one of the preceding claims configured to determine a vital sign of the subject from said detection signal.

12. System as claimed in claim 11,
wherein the stimulation unit (10) is configured to stimulate the skin region to cause vasodilation by a bio-thermal, bio-chemical, and/or photo-biological mechanism, in particular by heat, light, electromagnetic radiation and/or fluid spray.

13. System as claimed in claim 11 or 12,
further comprising an actuator (50) configured to change the direction and/or location of the stimulation and/or a thermal sensor (70) configured to monitor one or more of the location of the stimulation, the intensity of the stimulation, the duration of the stimulation and the skin temperature of the stimulated skin region.

14. Method for determining a vital sign of a subject, said method comprising:
- obtaining a detection signal derived from contactlessly detected electromagnetic radiation transmitted through or reflected from a skin region of a subject,
- determining a vital sign from said detection signal, and
- generating a control signal for controlling a stimulation unit configured to locally stimulate a skin region of the subject to cause vasodilation of the stimulated skin region.

15. Computer program comprising program code means for causing a computer to carry out the steps of the method as claimed in claim 14 when said computer program is carried out on the computer.
